# EUROPEAN PATENT SPECIFICATION

(11) **EP 3 434 238 B1**
(45) Date of publication and mention of the grant of the patent: **10.03.2021**
(21) Application number: 17182763.7
(22) Date of filing: 24.07.2017
(51) Int. Cl.: A61F 13/49, A61F 13/493

(54) **ABSORBENT ARTICLE FOR MULTIPLE SIZE COMFORTABLE FIT**
ABSORBIERENDER ARTIKEL FÜR BEQUEME PASSUNG FÜR MEHRERE GRÖSSEN
ARTICLE ABSORBANT POUR DE MULTIPLES DIMENSIONS CONFORTABLE

(43) Date of publication of application: 30.01.2019
(73) Proprietor: Ontex BV, 9255 Buggenhout (BE); Ontex Group NV, 9320 Erembodegem (BE)
(72) Inventor: Montepara, Paola, 66036 Orsogna (IT); Strazzella, Daniela, 66023 Francavilla al Mare (IT); De Poorter, Annick, 9320 Aalst-Erembodegem (BE)
(74) Representative: Larangé, Françoise

(56) References cited:
- EP-A2- 0 627 210
- WO-A1-98/56328
- US-A1- 2012 310 193
- US-A1- 2013 158 499

## Description

### TECHNICAL FIELD

The invention pertains to the technical field of absorbent hygiene products. In particular, the present invention relates to an absorbent article for absorbing body fluids and exudates, such as urine and fecal material. More particularly, the present invention relates to one-size-fits-all absorbent garments, such as disposable incontinence diapers, which are configured to collect and contain body fluids and exudates and avoid leakage.

### BACKGROUND

Infants and other incontinent individuals wear absorbent articles such as diapers to receive and contain urine and other body exudates. Absorbent articles function both to contain the discharged materials and to isolate these materials from the body of the wearer and from the wearer's garments and bed clothing. Disposable absorbent articles having many different basic designs are known to the art. For example, U.S. Patent Re. 26,152, entitled "Disposable Diaper" issued to Duncan and Baker on January 31, 1967, and U.S. Patent 3,860,003, entitled "Contractable Side Portions For Disposable Diaper", issued to Buell on January 14, 1975. However, absorbent articles have a tendency to sag or gap away from and to slide/slip down on the body of the wearer during wear. This sagging/gapping and sliding/slipping is caused by the relative motions of the wearer as the wearer breathes, moves and changes positions, by the downward forces generated when the absorbent article is loaded with body exudates, and by the rib-like element of the materials of the absorbent article itself when subjected to such wearer's motions. This sagging/gapping and sliding/slipping of the absorbent article can lead to premature leakage and poor fit of the absorbent article about the wearer in the waist regions and the leg regions of the absorbent article.

In order to more snugly fit absorbent articles about the wearer, certain commercially available absorbent articles have been provided with elastic features. An example of a disposable diaper with elastic side panels is disclosed in U.S. Patent 5,151,092, entitled "Absorbent Article With Dynamic Elastic Waist Feature Having Predisposed Flexural Hinge" issued to Buell, Clear, and Falcone on September 22, 1992. However, elastics are costly and require a certain degree of manipulation and handling during assembly. Further, while elastics do provide a degree of stretch for the absorbent article, the components of the absorbent article to which the elastics are attached are typically not elastic such that the elastics must be prestretched prior to being secured to the absorbent article or the inelastic components must be subjected to mechanical stretching (e.g., ring rolling) to enable the added elastic to be effective. Otherwise, the added elastic is restrained by the inelastic components.

Attempts to address such drawbacks have been made in several subsequent designs such as described in EP0748197A1, wherein a relatively low cost, easy to manufacture, absorbent article having sustained dynamic fit about the wearer during use is described providing an extensible waist feature on an absorbent article that exhibits an "elastic-like" behavior in the direction of applied force or elongation without the use of additional elastic material.

Further attempts to improve fit are described for example in EP0802778A2 and relating to a disposable diaper comprising an absorbent part, a pair of ear parts projecting in opposite directions from the opposite side edges of one longitudinal end portion of the absorbent part, respectively, and two fastening means attached to the side edges of the ear parts, respectively. Each side edge has a first side edge section and a second side edge section, each ear part has a stress relaxing structure, in which a tensile stress smaller than that which is induced in the peripheral portion of the ear part is induced, in a portion thereof other than the peripheral portion, and each fastening means is attached to the ear part in a pulling section of the side edge, overlapping at least part of a first side edge section and part of a second side edge section so that component tensile forces of a tensile force applied to the fastening means are distributed at a desired distribution ratio to the waist lapping portion and the leg lapping portion of the absorbent part.

Other attempts to improve fit focused on the combination of elastic side panels (or ears) with elastic waistband portions such as described in WO2009157835

US2013/0158499 discloses a diaper comprising a plurality of fasteners provided along the inner surface of the diaper to allow for adjustment of the rise of the diaper so that the diaper may fit various-sized individuals.

Although considerable attempts have been made to improve the overall fit and leakage prevention of such absorbent articles, there is still un unmet need to provide absorbent articles, and in particular adult incontinent diapers, that not only provide such improved fit and resistance to leakage but that further may accommodate multiple subject sizes with a single product.

Indeed, one of the complexities of the industry is that multiple designs of the same product are generally made in order to accommodate different subject sizes. It is highly desirable to be able to offer a cost-effective single product and a single design that may fit different subjects, i.e. a true "one-size-fits-all" product. Such standardization leads to advantages such as reduced production complexity and cost, reduced logistics and supply chain management complexities and thus all contributing to a cheaper whilst effective product for the end target user.

These and other objects of the present invention will be more readily apparent when considered in reference to the following description and when taken in conjunction with the accompanying drawings.

### SUMMARY OF THE INVENTION

In a first aspect, the present invention relates to an absorbent article, having a longitudinal axis and a transverse axis and comprising:
a chassis including a skin-facing side, a garment-facing side opposite to said skin-facing side, a front waist region proximal to a chassis front end, a back waist region proximal to a chassis back end and a crotch region extending between said front and back waist regions; a liquid-absorbent member extending across the crotch region towards the front and back waist regions, and having a front end and a back end; at least two oppositely disposed elastic rear panels joined to said chassis in said back waist region and extending laterally outward therefrom in a direction parallel to said transverse axis; and one or more fasteners positioned on each of said elastic rear panels proximal to an end thereof being distal from said chassis; wherein the front waist region of the chassis comprises a length adjustment region that is adapted such to vary the overall length of the absorbent article and wherein the distance between said front end and said chassis front end is greater than the distance between said back end and said chassis back end.

In a second aspect, the present invention relates to a method of changing the size of an absorbent article comprising the steps of: providing an absorbent article as described herein; adjusting the length of the length adjustment region to provide the best fit over the crotch region of a subject, preferably by folding said length adjustment region substantially on itself in such that a skin-facing surface of said region proximal to the chassis front end is in contact with a skin-facing surface of the chassis at a position distal from said chassis front end to form an overlap region having an overlap length extending parallel to the longitudinal axis said overlap length not exceeding the distance between the front end of the liquid-absorbent member and the chassis front end; and fastening the one or more fasteners to one or more locations of a garment facing side of the length adjustment region, preferably at a position substantially proximal to said fold.

### DESCRIPTION OF FIGURES

**Fig. 1** is a schematic plan view of a stretched and laid flat article according to an embodiment of the present disclosure.
**Fig. 2** (A and B) is a schematic plan view of the core used in an article according to an embodiment of the present disclosure.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or openended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, and the like. Absorbent articles preferably comprise a longitudinal axis and a transversal axis perpendicular to said longitudinal axis. The longitudinal axis is hereby conventionally chosen in the front-to-back direction of the article when referring to the article being worn.

"One-size-fits-all" refers to absorbent articles that exhibit size adjustment properties in both a first adjustment direction substantially parallel to a transversal axis X-X as well as a second adjustment direction substantially parallel to a longitudinal direction Y-Y, such that the same absorbent article may fit a plurality of different sized subjects. Typically the one-size-fits-all comprises at least two, preferably at least three, more preferably at least four, absorbent article sizes in a single article (preferably the sizes being selected from the group consisting of small, medium, large, and/or extra-large).

When used herein, reference to a "front" portion refers to that part of the diaper which is generally located on the front of an incontinent person when in use. Reference to the "rear" portion refers to the portion of the diaper generally located at the rear of the incontinent person when in use, and reference to the "crotch" portion refers to that portion which is generally located between the legs of an incontinent person when in use. The crotch region is an area where repeated fluid surge typically occurs.

Preferably, an absorbent article (e.g. a diaper) comprises a liquid permeable "top sheet", a liquid impermeable "back sheet", and an "absorbent medium" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sub-layer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many diapers, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present invention can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present invention. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

As used therein, the term "associated" encompasses configurations in which top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The terms "back section" and "rear back section" are used herein as synonyms and refer to the area of the absorbent article which is contact with the back of the wearer when the absorbent article is worn.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The terms "belly section" and "front belly section" are used herein as synonyms and refer to the area of the absorbent article which is contact with the belly of the wearer when the absorbent article is worn.

The term "blend" means a mixture of two or more polymers while the term "alloy" means a subclass of blends wherein the components are immiscible but have been compatibilized.

As used herein, the "body-facing" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to films having a water vapor transmission rate (WVTR) of at least 300 grams/m² - 24 hours.

"Carded web" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Chassis" refers to a foundational constituent of an absorbent article upon which the remainder of the structure of the article is built up or overlaid, e.g., in a diaper, the structural elements that give the diaper the form of briefs or pants when configured for wearing, such as a back sheet, a top sheet, or a combination of a top sheet and a back sheet.

"Coform" as used herein is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent particles. The meltblown fibers containing wood fibers are collected on a forming surface, such as provided by a foraminous belt. The forming surface may include a gas-pervious material, such as spunbonded fabric material, that has been placed onto the forming surface.

"Compression" refers to the process or result of pressing by applying force on an object, thereby increasing the density of the object.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, the diaper can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

The diaper can comprise leg containment gaskets. Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Continuous" means that the described structure is a closed-loop structure. The continuous structure may be unitary, i.e., a one-piece structure, or may be made up of individual elements suitably joined together to form a closed-loop.

A "continuous waistband" can be an elastomeric, cloth-like, nonwoven fibrous material, such as an elastomeric stretch bonded laminate web or an elastomeric meltblown web. By proper selection of materials, the continuous waistband can be rendered temporarily elastically inhibited, such as by compression. Once temporarily elastically inhibited, the elastic material, of which waistband is comprised, can be activated, such as by treating with heat, to recover to a state of elasticity.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "density" or "concentration" when referring to the absorbent material, in particular the SAP, of a layer, refers to the amount of the absorbent material divided by the surface area of the layer over which the absorbent material is spread out.

As used herein, the term "diaper" refers to an absorbent article generally worn by incontinent persons about the lower torso.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "elastic resistance" describes an elastic force that tends to resist an applied tensile force causing a material provided therewith to tend to contract to an untensioned configuration in response to a stretching force.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent). "Extension" means the change in length of a material due to stretching (expressed in units of length).

As used herein the term "extensible" means elongatable in at least one direction, but not necessarily recoverable.

The term "fabrics" is used to refer to all of the woven, knitted and nonwoven fibrous webs.

The term "finished" or "final", when used with reference to a product, means that the product has been suitably manufactured for its intended purpose.

The term "flexible" refers to materials which are compliant and which will readily conform to the general shape and contours of the wearer's body.

As used herein, the term "garment" means any type of apparel which may be worn. This includes diapers, training pants, incontinence products, surgical gowns, industrial workwear and coveralls, undergarments, pants, shirts, jackets and the like.

The term "graphic" includes, but is not limited to, any type of design, image, mark, figure, codes, words, patterns, or the like. For a product such as a training pant, graphics will generally include objects associated with little boys and little girls, such as multi-color trucks, airplanes, balls, dolls, bows, or the like.

The term "high-absorbency material" refers to materials that are capable of absorbing at least 10 times their own weight in liquid. The high-absorbency material may comprise absorbent gelling materials, such as superabsorbent polymers. Superabsorbent polymers are water-swellable, water-insoluble organic or inorganic materials capable of absorbing at least about 20 times their own weight of an aqueous solution containing 0.9 weight percent of sodium chloride. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces. Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alphaolefins, poly(vinyl pyrrolidone), poly(vinylmorpholinone), poly(vinyl alcohol), and mixtures and copolymers thereof. Further polymers suitable for use in the absorbent structure include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be used. Synthetic absorbent gelling materials typically are xerogels which form hydrogels when wetted. The term "hydrogel", however, has commonly been used to also refer to both the wetted and unwetted forms of the material. The high-absorbency material may be in any of a wide variety of geometric forms. As a general rule, it is preferred that the high-absorbency material be in the form of discrete particles. However, the high-absorbency material may also be in the form of fibres, flakes, rods, spheres, needles, spiral or semi-spiral, cubic, rod-like, polyhedral, or the like. Conglomerates of particles of high-absorbency material may also be used. The high-absorbency material may be present in the absorbent core in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Integral" is used to refer to various portions of a single unitary element rather than separate structures bonded to or placed with or placed near one another.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" is intended to refer to the article when it is flattened into a plane or is substantially flattened into a plane and is used in contrast to when the article otherwise positioned, such as when the article is folded or shaped in or for use by a wearer. Unless otherwise indicated herein, all measurements of the article described herein are taken in this condition.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

"Liquid" means a nongaseous substance and/or material that flows and can assume the interior shape of a container into which it is poured or placed.

"Longitudinal" is a direction running parallel to the maximum linear dimension of the article.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" refers to any material which does not fall within the definition of "elastic" above.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp fluff" or "fluff pulp" refers to a material made up of cellulose fibers. The fibers can be either natural or synthetic, or a combination thereof. The material is typically lightweight and has absorbent properties.

As used herein the term "sheet" or "sheet material" refers to woven materials, nonwoven webs, polymeric films, polymeric scrim-like materials, and polymeric foam sheeting.

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. "Stretch" may be unidirectional, bidirectional, or multi- directional. The specific "stretch" properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

Use of the term "substrate" includes, but is not limited to, woven or nonwoven webs, porous films, ink permeable films, paper, composite structures, or the like.

Superabsorbent materials suitable for use in the present invention are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCI). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight. Typically, the superabsorbent material, when present, will be included in an amount of about 5% to about 40% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighboring particles.

Embodiments of the articles according to the disclosure will now be described. It is understood that technical features described in one or more embodiments maybe combined with one or more other embodiments without departing from the intention of the disclosure and without generalization therefrom.

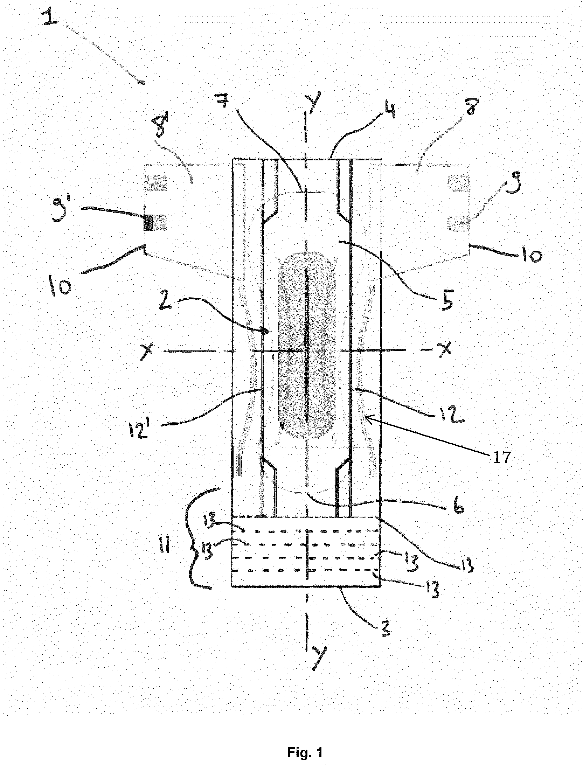

In a first aspect and with reference to Fig. 1, the disclosure herein relates to an absorbent article **1,** preferably an incontinence diaper, having a longitudinal axis **Y-Y** and a transverse axis **X-X** and comprising: a chassis **2** including a skin-facing side, a garment-facing side opposite to said skin-facing side, a front waist region proximal to a chassis front end **3,** a back waist region proximal to a chassis back end **4** and a crotch region extending between said front and back waist regions; a liquid-absorbent member **5** extending across the crotch region towards the front and back waist regions, and having a front end **6** and a back end **7;** at least two oppositely disposed elastic rear panels **8, 8'** joined to said chassis **2** in said back waist region [typically said panels not being integral with the chassis] and extending laterally outward therefrom in a direction parallel to said transverse axis **X-X;** and one or more fasteners **9,9'** positioned on each of said elastic rear panels **8, 8'** proximal to an end **10** thereof being distal from said chassis **2;** wherein the front waist region of the chassis **2** comprises a length adjustment region **11** that is adapted such to vary the overall length of the absorbent article **1** (typically in a direction parallel to the longitudinal axis **Y-Y**) and wherein the distance between said front end **6** and said chassis front end **3** is greater than the distance between said back end **7** and said chassis back end **4,** said distance typically taken along an axis parallel to the longitudinal axis Y-Y. An advantage of this embodiment is that the article may be adjusted both in length and width in order to accommodate different user sizes in a simple and cost effective manner.

In an embodiment (not shown), the absorbent article may further comprise a liquid permeable (preferably hydrophilic) topsheet and a liquid impermeable (preferably hydrophobic) backsheet, and the liquid absorbent member may be disposed therebetween. The topsheet may be positioned proximal to a skin facing side of the article, and the backsheet may be positioned proximal to a garment facing side of the article. Preferably, the backsheet is breathable and comprises a film material typically comprising polyethylene. The above mentioned layers may be joined together by use of a hotmelt adhesive. Optionally, the article may further comprise an acquisition distribution layer (ADL) positioned between the topsheet and the absorbent member.

In a preferred embodiment, the absorbent articles herein are free of front side panels in the front waist region such that in said front waist region lateral edges (extending parallel to the longitudinal axis Y-Y) thereof correspond to outermost lateral edges of the chassis. Typically, the chassis being substantially rectangular in shape.

In an embodiment, the absorbent article **1** further comprises oppositely disposed longitudinal barriers **12,12'** extending parallel to the longitudinal axis **Y-Y** and extending along the length of said absorbent article **1,** preferably said barriers **12,12'** comprising one or more elastics such that, when worn by a subject, said barriers **12,12'** may stand vertically in a direction perpendicular to the longitudinal axis **Y-Y** and the transverse axis **X-X** and parallel to a thickness direction of the absorbent article **1.** This arrangement has the advantage of further leakage prevention in the lateral directions.

Preferably, the longitudinal barriers **12,12'** are joined to the chassis **2** along a longitudinal length thereof along a joining area and wherein at least a portion of said barriers **12,12'** is not joined to the chassis **2** to form a non-joining area, and wherein the non-joining area is proximal to the chassis front end **3** and/or to the chassis back end **4** such to form one or more pockets for retaining exudates therein. An advantage of this arrangement is to further generate transversal barrier surfaces without adding further materials or components to the absorbent article and thus reducing making process complexity as well as cost.

In an embodiment, the length adjustment region **11** is arranged to fold substantially on itself at a plurality of positions such that a skin-facing surface of said region **11** proximal to the chassis front end **3** is in contact with a skin-facing surface of the chassis **2** at a position distal from said chassis front end **3** to form an overlap region having an overlap length extending parallel to the longitudinal axis **Y-Y** said overlap length not exceeding the distance between the front end **6** of the liquid-absorbent member **5** and the chassis front end **3.** An advantage of this arrangement is that the fold itself may behave as a transversal frontal barrier to trap and further prevent exudates from leaking through the frontal portion of the article.

Preferably, said plurality of positions at least comprise a first position and a second position wherein the overlap length in the first position is greater than the overlap length in the second position so that the total length of the absorbent article along the longitudinal axis **Y-Y** in the first position is less than the said total length in the second position, such that when the absorbent article **1** is worn by a subject, said second position may accommodate a larger subject than said first position.

In an embodiment, the length adjustment region **11** comprises a plurality of folding lines **13** each corresponding to one of the plurality of positions, preferably wherein said plurality of folding lines **13** are spaced apart and located at predetermined distances along the longitudinal axis **Y-Y,** and preferably correspond to a predetermined size of absorbent article. An advantage of this arrangement is that a series of reproducible and pre-set dimension and sizing can be formed so that the user can reliably choose a consistent setting to be used based on his or her size.

Preferably, the overlap region forms a transversal barrier when the absorbent article is worn by a subject, said transversal barrier extending through an entire width of the chassis **2** extending along the transverse axis **X-X.** An advantage of this arrangement is that a transversal barrier is formed that truly extends throughout the totality of the chassis width.

In a embodiment, the fasteners **9,9'** are arranged to engage with the garment facing side of the length adjustment region **11.** Preferably wherein the fasteners **9,9'** comprise, or consist of, mechanical fasteners typically having one or more hook regions arranged to mechanically engage with a respective target surface, of a garment facing side of the article, comprising a nonwoven or knitted surface.

In an embodiment, the outermost surface on the garment facing side of at least the length adjustment region **11** of the chassis **2** comprises a nonwoven material, preferably comprising, or consisting of, a spunbond nonwoven typically having a basis weight of from 5 gsm to 25 gsm, preferably from 8 gsm to 20 gsm, most preferably from 9 gsm to 14 gsm. Preferably, said nonwovens comprise an embossed pattern, preferably a pillow-shaped pattern. Advantageously, specifically selecting such nonwovens enables increased comfort and softness when the article is worn by smaller sized subjects. Moreover, adding an embossment also works synergistically with the fasteners, especially when the fasteners comprise or consist of mechanical fasteners as described herein, by creating a 3-D profile onto which the hooks of the fasteners may more effectively engage with, thus providing a stronger closing force as well as providing the functional and perceived softness to the subject.

In an embodiment, the elastic rear panels **8, 8'** are arranged to provide an elongation of at least 50%, preferably from 50% to 250%, more preferably from 60% to 200%, more preferably from 70% to 150%. Such has the advantage of accommodating different circumferential sizes and ensuring a good and snug fit of the product. Preferably, the elastic rear panels are joined to the chassis by a pressure sensitive adhesive or by mechanical bonding such as ultrasonic bonding.

In an embodiment at least a portion of the length adjustment region **11** comprises an elastic web material, preferably wherein the chassis **2** is substantially non-elastic except for said length adjustment region **11.** An advantage of this arrangement is that further sizes may be incorporated into the single article. Moreover, the user may then choose whether to still fold said region to form the frontal barrier (by stretching and folding it in place prior to fastening) or to fasten the article without frontal barrier, thus allowing more construction choices based on the needs and with the same one article.

Preferably, the length adjustment region **11** comprises indicia arranged such that for each position of a plurality of positions, a corresponding size of a subject can be distinguished. Such permits consistency with other product ranges and sizes such that a user used to a given size would immediately recognize the setting to be used in the articles described herein. The "indica" may be in the form of one or more colors, graphics, numbers or letters.

In an embodiment, the distance ratio, of the distance between said front end **6** and said chassis front end **3** over the distance between said back end **7** and said chassis back end **4,** is greater than 2.0, preferably from 2.5 to 9.0, more preferably from 2.7 to 8.0, even more preferably from 2.8 to 5.5, most preferably from 2.9 to 3.5. Such arrangement is found beneficial for ensuring correct positioning of the absorbent core in the one-size-fits-all arrangement as well as providing sufficient material in the front portion of the article to provide for the useful size adjustments.

In an embodiment, the liquid-absorbent member **5** comprises a first core **14** and a second core **15** disposed over the first core **14** and arranged such that the second core **15** is proximal to a skin facing side of the chassis **2** and the first core **14** is proximal to a garment facing side of said chassis **2** and distal from said skin facing side, wherein the first core **14** comprises one or more channels **16,** and preferably wherein the second core **15** is free of one or more channels. An advantage of this arrangement is improved liquid distribution and absorption over the entire core surface.

Preferably, the first core **14** has a substantially hour glass shape and the shape of the second core **15** is different from the shape of the first core **14.** Typically wherein the second core covers less than 65%, preferably 60%, of the surface area of the first core.

The first core **14** and the second core **15** may each comprise a combination of both a high absorbency material (like superabsorbent polymer particles (SAP)) and fibrous absorbent material (such as cellulose fibers), wherein the ratio of SAP:fibrous material in the first core is different from said ratio in the second core, preferably wherein said ratio is greater in the first core than in the second core. In a preferred embodiment, the SAP density in the first core is greater than the SAP density in the second core, more preferably at least 1.5 or 2.0 times greater.

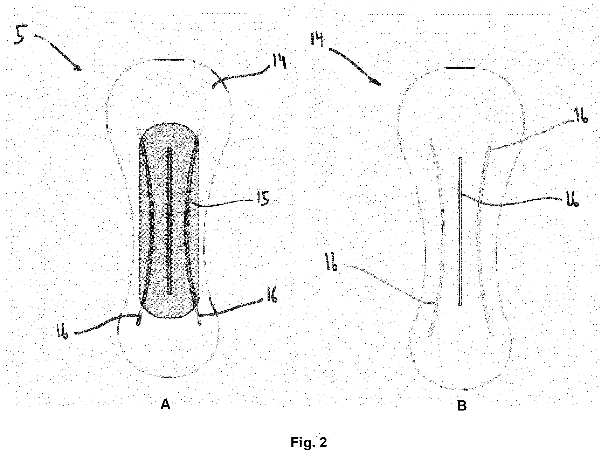

In a preferred embodiment and as shown in Fig. 2, the first core **14** comprises at least three channels, a central substantially linear channel and at least one arcuate channel disposed on each opposite side of the central channel. The channels may extend along the longitudinal axis **Y-Y,** and each arcuate channels may comprise a convex surface and a concave surface, and each arcuate channel is disposed such that the convex surface thereof faces the central linear channel. An advantage of this arrangement is not only to maximize liquid distribution across the core but also to provide folding lines for better shaping and comfort, especially beneficial in a one-size-fits-all arrangement.

In an embodiment, the absorbent article **1** may further comprise one or more leg elastics **17** on each lateral side outboard of the liquid-absorbent member **5,** preferably the leg elastics being nonlinear. Typically wherein at least three leg elastics **17** are present on each said lateral side. An advantage of this embodiment is that further comfort is enabled as well as a good fir around the user legs.

In an embodiment, the method of changing the size of an absorbent article as described herein comprises the steps of: adjusting the length of the length adjustment region **11** to provide the best fit over the crotch region of a subject, preferably by folding said length adjustment region **11** substantially on itself such that a skin-facing surface of said region **11** proximal to the chassis front end **3** is in contact with a skin-facing surface of the chassis **2** at a position distal from said chassis front end **3** to form an overlap region having an overlap length extending parallel to the longitudinal axis **Y-Y** said overlap length not exceeding the distance between the front end **6** of the liquid-absorbent member **5** and the chassis front end **3;** and fastening the one or more fasteners **9,9'** to one or more locations of a garment facing side of the length adjustment region **11,** preferably at a position substantially proximal to said fold. In this simple and effective manner, the absorbent articles described herein may be adjusted to fit multiple user sizes, without the need to produce or purchase different product designs.

### Method of measuring elongation

This test method is designed to determine the elasticity of elastic web materials in inco pants to ensure usability of the pants. The pants are placed in a mounting frame and at predefined forces the percentage of elongation is determined.
**Material:** Tensile tester [like a Zwick / Roell Z1.0] with constant rate of extension calibrated according to the manufacturer's instructions; Clamps and jaws: each jaw face shall be smooth, flat and with a metallic surface and dimension of 30 x 60 mm. The faces shall be parallel and have matching centres with respect to one another in the same clamp and to the corresponding jaw face of the other clamp; Mounting frame: includes a top & bottom device to be placed between the clamps.
**Procedure:** The diapers are placed over a mounting frame on the tensile testing machine and pulled with constant rate of extension. Values for elongation of the test products are obtained from a computer interface.
**Definitions:** Elongation: the deformation in the direction of load caused by a tensile force. Elongation is generally expressed as a percentage of the length of the stretched material to the length of the un-stretched material (= distance between frame-ends at the beginning of the test - LE); Extension: the change of length of a material due to stretching.
**Equipment verification:** Verify load cell: a calibrated verification weight of 500g should give a force of 4.90(5) N ± 0.10 N.
**Equipment Parameters:** Following parameters are programmed: Pre-load : 0,1N; Cross Head Speed : 300 mm/min; End of Test: 22N
Gage Length LE (mm) needs to be adjusted depending on the waist size of the diaper (from 220mm in its smallest configuration and 270mm in its maximized size.
**Procedure:** Assemble the mounting frame & zero the load cell; Adjust the gage length according to the waist size; Carefully place the diaper over the mounting frame; Zero the load cell; Start the test Force (N) and test path (mm) measurements will be recorded until 22N force is reached; Repeat for at least 5 products.
**Calculation:** Record the elongation value at standard setting forces 2N and 20N for each product. Measure the increase in length from the start of the force-extension curve to the point corresponding with the predefined force. Calculate the elongation as the percentage increase in length based on the gage length for both 2N and 20N.

It is supposed that the present invention is not restricted to any form of realization described previously and that some modifications can be added to the presented example of fabrication without reappraisal of the appended claims.

## Claims

1. An absorbent article (1), preferably an incontinence diaper, having a longitudinal axis (Y-Y) and a transverse axis (X-X) and comprising:
a chassis (2) including a skin-facing side, a garment-facing side opposite to said skin-facing side, a front waist region proximal to a chassis front end (3), a back waist region proximal to a chassis back end (4) and a crotch region extending between said front and back waist regions;
a liquid-absorbent member (5) extending across the crotch region towards the front and back waist regions, and having a front end (6) and a back end (7);
at least two oppositely disposed elastic rear panels (8, 8') joined to said chassis (2) in said back waist region and extending laterally outward therefrom in a direction parallel to said transverse axis (X-X); and
one or more fasteners (9,9') positioned on each of said elastic rear panels (8, 8') proximal to an end (10) thereof being distal from said chassis (2);
**characterized in that** the front waist region of the chassis (2) comprises a length adjustment region (11) that is adapted such to vary the overall length of the absorbent article (1) and **in that** the distance between said front end (6) and said chassis front end (3) is greater than the distance between said back end (7) and said chassis back end (4).

2. An absorbent article (1) according to Claim 1 further comprising oppositely disposed longitudinal barriers (12,12') extending parallel to the longitudinal axis (Y-Y) and extending along the length of said absorbent article (1), preferably said barriers (12,12') comprising one or more elastics such that, when worn by a subject, said barriers (12,12') may stand vertically in a direction perpendicular to the longitudinal axis (Y-Y) and the transverse axis (X-X) and parallel to a thickness direction of the absorbent article (1).

3. An absorbent article (1) according to Claim 2 wherein the longitudinal barriers (12,12') are joined to the chassis (2) along a longitudinal length thereof along a joining area and wherein at least a portion of said barriers (12,12') is not joined to the chassis (2) to form a non-joining area, and wherein the non-joining area is proximal to the chassis front end (3) and/or to the chassis back end (4) such to form one or more pockets for retaining exudates therein.

4. An absorbent article (1) according to any of the preceding Claims wherein the length adjustment region (11) is arranged to fold substantially on itself in a plurality of positions such that a skin-facing surface of said region (11) proximal to the chassis front end (3) is in contact with a skin-facing surface of the chassis (2) at a position distal from said chassis front end (3) to form an overlap region having an overlap length extending parallel to the longitudinal axis (Y-Y) said overlap length not exceeding the distance between the front end (6) of the liquid-absorbent member (5) and the chassis front end (3).

5. An absorbent article (1) according to Claim 4 wherein said plurality of positions at least comprise a first position and a second position wherein the overlap length in the first position is greater than the overlap length in the second position so that the total length of the absorbent article along the longitudinal axis (Y-Y) in the first position is less than the said total length in the second position, such that when the absorbent article (1) is worn by a subject, said second position may accommodate a larger subject than said first position.

6. An absorbent article (1) according to Claims 4 to 5 wherein the length adjustment region (11) comprises a plurality of folding lines (13) each corresponding to one of the plurality of positions, preferably wherein said plurality of folding lines (13) are spaced apart and located at predetermined distances along the longitudinal axis (Y-Y).

7. An absorbent article (1) according to Claims 4 to 6 wherein the overlap region forms a transversal barrier when the absorbent article is worn by a subject, said transversal barrier extending through an entire width of the chassis (2) extending along the transverse axis (X-X).

8. An absorbent article (1) according to any of the preceding Claims wherein the fasteners (9,9') are arranged to engage with the garment facing side of the length adjustment region (11).

9. An absorbent article (1) according to any of the preceding Claims wherein an outermost surface on the garment facing side of at least the length adjustment region (11) of the chassis (2) comprises a nonwoven material, preferably comprising, or consisting of, a spunbond nonwoven typically having a basis weight of from 5 gsm to 25 gsm, preferably from 8 gsm to_20 gsm.

10. An absorbent article (1) according to any of the preceding Claims wherein the elastic rear panels (8, 8') are arranged to provide an elongation of at least 50%, preferably from 50% to 250%, more preferably from 60% to 200%, more preferably from 70% to 150%.

11. An absorbent article (1) according to any of the preceding Claims wherein at least a portion of the length adjustment region (11) comprises an elastic web material, preferably wherein the chassis (2) is substantially non-elastic except for said length adjustment region (11).

12. An absorbent article (1) according to any of the preceding Claims wherein the length adjustment region (11) comprises indicia arranged such that for each position of a plurality of positions, a corresponding size of a subject can be distinguished.

13. An absorbent article (1) according to any of the preceding Claims wherein the distance ratio, of the distance between said front end (6) and said chassis front end (3) over the distance between said back end (7) and said chassis back end (4), is greater than 2.0, preferably from 2.5 to 9.0, more preferably from 2.7 to 8.0, even more preferably from 2.8 to 5.5, most preferably from 2.9 to 3.5.

14. An absorbent article (1) according to any of the preceding Claims wherein the liquid-absorbent member (5) comprises a first core (14) and a second core (15) disposed over the first core (14) and arranged such that the second core (15) is proximal to a skin facing side of the chassis (2) and the first core (14) is proximal to a garment facing side of said chassis (2) and distal from said skin facing side, wherein the first core (14) comprises one or more channels (16), and preferably wherein the second core (15) is free of one or more channels.

15. A method of changing the size of an absorbent article comprising the steps of:
providing an absorbent article (1) according to any of the preceding Claims;
adjusting the length of the length adjustment region (11) to provide the best fit over the crotch region of a subject, preferably by folding said length adjustment region (11) substantially on itself such that a skin-facing surface of said region (11) proximal to the chassis front end (3) is in contact with a skin-facing surface of the chassis (2) at a position distal from said chassis front end (3) to form an overlap region having an overlap length extending parallel to the longitudinal axis (Y-Y) said overlap length not exceeding the distance between the front end (6) of the liquid-absorbent member (5) and the chassis front end (3); and
fastening the one or more fasteners (9,9') to one or more locations of a garment facing side of the length adjustment region (11), preferably at a position substantially proximal to said fold.

## Patentansprüche

1. Absorbierender Gegenstand (1), vorzugsweise Inkontinenzwindel, mit einer Längsachse (Y-Y) und einer Querachse (X-X) und umfassend:
einen Körper (2), der eine der Haut zugewandte Seite, eine der Kleidung zugewandte Seite gegenüber der der Haut zugewandten Seite, eine vordere Taillenregion proximal zu einem vorderen Körper-Ende (3), eine hintere Taillenregion (4) proximal zu einem hinteren Körper-Ende und eine Schrittregion umfasst, die sich zwischen der vorderen und der hinteren Taillenregion erstreckt;
ein Flüssigkeit absorbierendes Element (5), das sich über die Schrittregion zur vorderen und hinteren Taillenregion erstreckt und ein vorderes Ende (6) und ein hinteres Ende (7) aufweist;
mindestens zwei gegenüberliegend angeordnete elastische hintere Stoffbahnen (8, 8'), die in der hinteren Taillenregion mit dem Körper (2) verbunden sind und sich in einer Richtung parallel zur Querachse (X-X) lateral davon nach außen erstrecken; und
einen oder mehrere Befestigungselemente (9,9'), die auf jeder der elastischen hinteren Stoffbahnen (8, 8') proximal zu einem Ende (10) davon positioniert sind, das distal vom Körper (2) ist;
**dadurch gekennzeichnet, dass** die vordere Taillenregion des Körpers (2) eine Längenanpassungsregion (11) umfasst, die derart ausgelegt ist, dass sie die Gesamtlänge des absorbierenden Gegenstands (1) ändert, und dadurch, dass der Abstand zwischen dem vorderen Ende (6) und dem vorderen Körper-Ende (3) größer als der Abstand zwischen dem hinteren Ende (7) und dem hinteren Körper-Ende (4) ist.

2. Absorbierender Gegenstand (1) nach Anspruch 1, ferner umfassend gegenüberliegend angeordnete Längsbarrieren (12,12'), die sich parallel zur Längsachse (Y-Y) erstrecken und entlang einer Länge des absorbierenden Gegenstands (1) verlaufen, wobei die Barrieren (12,12') vorzugsweise ein oder mehrere Gummibänder umfassen, derart dass, wenn von einem Individuum getragen, die Barrieren (12,12') vertikal in einer Richtung senkrecht zur Längsachse (Y-Y) und der Querachse (X-X) und parallel zur einer Dickenrichtung des absorbierenden Gegenstands (1) stehen können.

3. Absorbierender Gegenstand (1) nach Anspruch 2, wobei die Längsbarrieren (12,12') mit dem Körper (2) entlang einer der längslaufenden Länge davon entlang eines Verbindungsbereichs verbunden sind, und wobei mindestens ein Abschnitt der Barrieren (12,12') nicht mit dem Körper (2) verbunden ist, um einen unverbundenen Bereich zu bilden, und wobei der unverbundene Bereich proximal zum vorderen Körper-Ende (3) und/oder zum hinteren Körper-Ende (4) ist, um eine oder mehrere Taschen zum Zurückhalten von Exsudaten darin zu bilden.

4. Absorbierender Gegenstand (1) nach einem der vorhergehenden Ansprüche, wobei die Längenanpassungsregion (11) so ausgelegt ist, dass sie sich in einer Mehrzahl von Positionen im Wesentlichen auf sich selbst faltet, derart dass eine der Haut zugewandte Fläche der Region (11) proximal zum vorderen Körper-Ende (3) in einer Position distal vom vorderen Körper-Ende (3) mit einer der Haut zugewandten Fläche des Körpers (2) in Kontakt ist, um eine Überlappungsregion mit einer Überlappungslänge zu bilden, die sich parallel zur Längsachse (Y-Y) erstreckt, wobei die Überlappungslänge den Abstand zwischen dem vorderen Ende (6) des Flüssigkeit absorbierenden Elements (5) und dem vorderen Körper-Ende (3) nicht überschreitet.

5. Absorbierender Gegenstand (1) nach Anspruch 4, wobei die Mehrzahl von Positionen mindestens eine erste Position und eine zweite Position umfasst, wobei die Überlappungslänge in der ersten Position größer als die Überlappungsregion in der zweiten Position ist, so dass die Gesamtlänge des absorbierenden Gegenstands entlang der Längsachse (Y-Y) in der ersten Position kürzer als die Gesamtlänge in der zweiten Position ist, derart dass, wenn der absorbierende Gegenstand (1) von einem Individuum getragen wird, die zweite Position ein größeres Individuum aufnehmen kann als die erste Position.

6. Absorbierender Gegenstand (1) nach Anspruch 4 oder 5, wobei die Längenanpassungsregion (11) eine Mehrzahl von Faltlinien (13) umfasst, die jeweils eine der Mehrzahl von Positionen entsprechen, wobei die Mehrzahl von Faltlinien (13) vorzugsweise voneinander beabstandet ist und in einem vorbestimmten Abstand entlang der Längsachse (Y-Y) angeordnet ist.

7. Absorbierender Gegenstand (1) nach Anspruch 4 bis 6, wobei die Überlappungsregion eine Querbarriere bildet, wenn der absorbierende Gegenstand von einem Individuum getragen wird, wobei die Querbarriere durch eine gesamte Breite des Körpers (2) verläuft, die sich entlang der Querachse (X-X) erstreckt.

8. Absorbierender Gegenstand (1) nach einem der vorhergehenden Ansprüche, wobei die Befestigungselemente (9, 9') so ausgelegt sind, dass sie mit der der Kleidung zugewandten Seite des Längenanpassungsregion (11) in Eingriff stehen.

9. Absorbierender Gegenstand (1) nach einem der vorhergehenden Ansprüche, wobei eine äußerste Fläche auf der der Kleidung zugewandten Seite zumindest der Längenanpassungsregion (11) des Körpers (2) ein Vliesmaterial umfasst, das vorzugweise ein spinngebundenes Vlies mit einem Flächengewicht von 5 gsm bis 25 gsm und vorzugsweise von 8 gsm bis 20 gsm umfasst oder daraus besteht.

10. Absorbierender Gegenstand (1) nach einem der vorhergehenden Ansprüche, wobei die elastischen hinteren Stoffbahnen (8, 8') so ausgelegt sind, dass sie eine Dehnung von mindestens 50 %, vorzugsweise von 50 % bis 250 %, insbesondere von 60 % bis 200 % und vornehmlich von 70 % bis 150 % bereitstellen.

11. Absorbierender Gegenstand (1) nach einem der vorhergehenden Ansprüche, wobei mindestens ein Abschnitt der Längenanpassungsregion (11) ein elastisches Bahnmaterial umfasst, wobei der Körper (2) mit Ausnahme der Längenanpassungsregion (11) vorzugsweise im Wesentlichen nicht elastisch ist.

12. Absorbierender Gegenstand (1) nach einem der vorhergehenden Ansprüche, wobei die Längenanpassungsregion (11) eine Markierung umfasst, die derart ausgelegt ist, dass für jede Position einer Mehrzahl von Positionen eine entsprechende Größe eines Individuums unterschieden werden kann.

13. Absorbierender Gegenstand (1) nach einem der vorhergehenden Ansprüche, wobei das Abstandsverhältnis des Abstands zwischen dem vorderen Ende (6) und dem vorderen Körper-Ende (3) gegenüber dem Abstand zwischen dem hinteren Ende (7) und dem hinteren Körper-Ende (4) größer als 2,0, vorzugsweise 2,5 bis 9,0, insbesondere 2,7 bis 8.0, vornehmlich 2,8 bis 5,5 und am besten 2,9 bis 3,5 ist.

14. Absorbierender Gegenstand (1) nach einem der vorhergehenden Ansprüche, wobei das Flüssigkeit absorbierende Element (5) einen ersten Kern (14) und einen zweiten Kern (15) umfasst, der über dem ersten Kern (14) angeordnet und derart ausgelegt ist, dass der zweite Kern (15) proximal zu einer der Haut zugewandten Seite des Körpers (2) ist, und der erste Kern (14) proximal zu einer der Kleidung zugewandten Seite des Körpers (2) und distal von der der Haut zugewandten Seite ist, wobei der erste Kern (14) einen oder mehrere Kanäle (16) umfasst, und wobei der zweite Kern (15) vorzugsweise frei von einem oder mehreren Kanälen ist.

15. Verfahren zur Änderung der Größe eines absorbierenden Gegenstands, umfassend die folgenden Schritte:
Bereitstellen eines absorbierenden Gegenstands (1) nach einem der vorhergehenden Ansprüche;
Anpassen der Länge der Längenanpassungsregion (11), um die beste Passung über die Schrittregion eines Individuums bereitzustellen, vorzugsweise durch Falten der Längenanpassungsregion (11) im Wesentlichen auf sich selbst, derart dass eine der Haut zugewandte Fläche der Region (11) proximal zum vorderen Körper-Ende (3) in einer Position distal vom vorderen Körper-Ende (3) mit einer der Haut zugewandten Fläche des Körpers (2) in Kontakt ist, um eine Überlappungsregion mit einer Überlappungslänge zu bilden, die sich parallel zur Längsachse (Y-Y) erstreckt, wobei die Überlappungslänge den Abstand zwischen dem vorderen Ende (6) des Flüssigkeit absorbierenden Elements (5) und dem vorderen Körper-Ende (3) nicht überschreitet; und
Befestigen des einen oder der mehreren Befestigungselemente (9,9') an einer oder mehreren Stellen einer der Kleidung zugewandten Seite der Längenanpassungsregion (11), vorzugsweise in einer Position, die im Wesentlichen proximal zur Faltung ist.

## Revendications

1. Article absorbant (1), de préférence une couche pour incontinence, ayant un axe longitudinal (Y-Y) et un axe transversal (X-X) et comprenant :
une structure (2) incluant un côté tourné vers la peau, un côté tourné vers le vêtement opposé audit côté tourné vers la peau, une région de taille avant proximale à une extrémité avant de structure (3), une région de taille arrière proximale à une extrémité arrière de structure (4) et une région d'entrejambe s'étendant entre lesdites régions de taille avant et arrière ;
un élément absorbant les liquides (5) s'étendant à travers la région d'entrejambe vers les régions de taille avant et arrière, et ayant une extrémité avant (6) et une extrémité arrière (7) ;
au moins deux panneaux arrière élastiques disposés de manière opposée (8, 8') reliés à ladite structure (2) dans ladite région de taille arrière et s'étendant latéralement vers l'extérieur à partir de celle-ci dans une direction parallèle audit axe transversal (X-X) ; et
une ou plusieurs fixations (9,9') positionnées sur chacun desdits panneaux arrière élastiques (8, 8') de façon proximale à une extrémité (10) de celui-ci sont distales par rapport à ladite structure (2) ;
**caractérisé en ce que** la région de taille avant de la structure (2) comprend une région d'ajustement de longueur (11) qui est conçue de sorte à faire varier la longueur totale de l'article absorbant (1) et **en ce que** la distance entre ladite extrémité avant (6) et ladite extrémité avant de structure (3) est supérieure à la distance entre ladite extrémité arrière (7) et ladite extrémité arrière de structure (4).

2. Article absorbant (1) selon la revendication 1, comprenant en outre des barrières longitudinales disposées de manière opposée (12, 12') s'étendant parallèlement à l'axe longitudinal (Y-Y) et s'étendant sur la longueur dudit article absorbant (1), de préférence lesdites barrières (12, 12') comprenant un ou plusieurs élastiques de telle sorte que, lorsqu'il est porté par un sujet, lesdites barrières (12, 12') peuvent se tenir verticalement dans une direction perpendiculaire à l'axe longitudinal (Y-Y) et à l'axe transversal (X-X) et parallèlement à une direction d'épaisseur de l'article absorbant (1).

3. Article absorbant (1) selon la revendication 2, dans lequel les barrières longitudinales (12, 12') sont reliées à la structure (2) le long d'une longueur longitudinale de celui-ci le long d'une zone de jonction et dans lequel au moins une partie desdites barrières (12, 12') n'est pas reliée à la structure (2) pour former une zone de non-jonction, et dans lequel la zone de non-jonction est proximale à l'extrémité avant de structure (3) et/ou à l'extrémité arrière de structure (4) de sorte à former une ou plusieurs poches pour retenir des exsudats à l'intérieur de celles-ci.

4. Article absorbant (1) selon l'une quelconque des revendications précédentes, dans lequel la région d'ajustement de longueur (11) est conçue pour se plier sensiblement sur elle-même dans une pluralité de positions de telle sorte qu'une surface tournée vers la peau de ladite région (11) proximale à l'extrémité avant de structure (3) est en contact avec une surface tournée vers la peau de structure (2) au niveau d'une position distale par rapport à ladite extrémité avant de structure (3) pour former une région de chevauchement ayant une longueur de chevauchement s'étendant parallèlement à l'axe longitudinal (Y-Y), ladite longueur de chevauchement ne dépassant pas la distance entre l'extrémité avant (6) de l'élément absorbant les liquides (5) et l'extrémité avant de structure (3).

5. Article absorbant (1) selon la revendication 4, dans lequel ladite pluralité de positions comprend au moins une première position et une seconde position, dans lequel la longueur de chevauchement dans la première position est supérieure à la longueur de chevauchement dans la seconde position, de telle sorte que la longueur totale de l'article absorbant le long de l'axe longitudinal (Y-Y) dans la première position est inférieure à ladite longueur totale dans la seconde position, de telle sorte que lorsque l'article absorbant (1) est porté par un sujet, ladite seconde position peut s'adapter à un sujet plus gros que ladite première position.

6. Article absorbant (1) selon les revendications 4 à 5, dans lequel la région d'ajustement de longueur (11) comprend une pluralité de lignes de pliage (13) correspondant chacune à l'une de la pluralité de positions, de préférence, dans lequel ladite pluralité de lignes de pliage (13) sont espacées et situées à des distances prédéterminées le long de l'axe longitudinal (Y-Y).

7. Article absorbant (1) selon les revendications 4 à 6, dans lequel la région de chevauchement forme une barrière transversale lorsque l'article absorbant est porté par un sujet, ladite barrière transversale s'étendant sur toute la largeur de la structure (2) s'étendant le long de l'axe transversal (X-X).

8. Article absorbant (1) selon l'une quelconque des revendications précédentes, dans lequel les fixations (9, 9') sont conçues pour venir en prise avec le côté tourné vers le vêtement de la région d'ajustement de longueur (11).

9. Article absorbant (1) selon l'une quelconque des revendications précédentes, dans lequel une surface la plus extérieure sur le côté tourné vers le vêtement d'au moins la région d'ajustement de longueur (11) de la structure (2) comprend un matériau non tissé, comprenant de préférence, ou consistant en, un non-tissé filé-lié ayant d'ordinaire un poids de base de 5 g/m² à 25 g/m², de préférence de 8 g/m² à 20 g/m².

10. Article absorbant (1) selon l'une quelconque des revendications précédentes, dans lequel les panneaux arrière élastiques (8, 8') sont conçus pour fournir un allongement d'au moins 50 %, de préférence de 50 % à 250 %, de manière davantage préférée de 60 % à 200 %, de manière davantage préférée de 70 % à 150 %.

11. Article absorbant (1) selon l'une quelconque des revendications précédentes, dans lequel au moins une partie de la région d'ajustement de longueur (11) comprend un matériau de bande élastique, de préférence dans lequel la structure (2) est sensiblement non élastique à l'exception de ladite région d'ajustement de longueur (11).

12. Article absorbant (1) selon l'une quelconque des revendications précédentes, dans lequel la région d'ajustement de longueur (11) comprend des repères conçus de telle sorte que pour chaque position d'une pluralité de positions, une taille correspondante d'un sujet peut être distinguée.

13. Article absorbant (1) selon l'une quelconque des revendications précédentes, dans lequel le rapport de distances, de la distance entre ladite extrémité avant (6) et ladite extrémité avant de structure (3) sur la distance entre ladite extrémité arrière (7) et ladite extrémité arrière de structure (4), est supérieur à 2,0, de préférence, de 2,5 à 9,0, de manière davantage préférée, de 2,7 à 8,0, de manière encore davantage préférée, de 2,8 à 5,5, de manière préférée entre toutes, de 2,9 à 3,5.

14. Article absorbant (1) selon l'une quelconque des revendications précédentes, dans lequel l'élément absorbant les liquides (5) comprend un premier noyau (14) et un second noyau (15) disposé sur le premier noyau (14) et conçu de telle sorte que le second noyau (15) est proximal à un côté tourné vers la peau de la structure (2) et le premier noyau (14) est proximal à un côté tourné vers le vêtement de ladite structure (2) et distal par rapport audit côté tourné vers la peau, dans lequel le premier noyau (14) comprend un ou plusieurs canaux (16), et de préférence, dans lequel le second noyau (15) est dépourvu d'un ou de plusieurs canaux.

15. Procédé de modification de la taille d'un article absorbant comprenant les étapes de :
fourniture d'un article absorbant (1) selon l'une quelconque des revendications précédentes ;
ajustement de la longueur de la région d'ajustement de longueur (11) pour fournir le meilleur ajustement sur la région d'entrejambe d'un sujet, de préférence par pliage de ladite région d'ajustement de longueur (11) sensiblement sur elle-même de telle sorte qu'une surface tournée vers la peau de ladite région (11) proximale à l'extrémité avant de structure (3) est en contact avec une surface tournée vers la peau de la structure (2) au niveau d'une position distale par rapport à ladite extrémité avant de structure (3) pour former une région de chevauchement ayant une longueur de chevauchement s'étendant parallèlement à l'axe longitudinal (Y-Y), ladite longueur de chevauchement ne dépassant pas la distance entre l'extrémité avant (6) de l'élément absorbant les liquides (5) et l'extrémité avant de structure (3) ; et
fixation de la ou des fixations (9, 9') à un ou plusieurs emplacements d'un côté tourné vers le vêtement de la région d'ajustement de longueur (11), de préférence au niveau d'une position sensiblement proximale audit pli.
